# EUROPEAN PATENT APPLICATION

(11) **EP 4 763 072 A1**
(43) Date of publication of application: **24.06.2026**
(21) Application number: 24222150.5
(22) Date of filing: 20.12.2024
(51) Int. Cl.: A61B 5/06, A61B 5/07, A61B 5/145, A61B 5/01

(54) **AN INGESTIBLE DEVICE, A SYSTEM AND A METHOD FOR ANALYSIS OF THE GASTROINTESTINAL TRACT AND A WEARABLE DEVICE FOR LOCALIZATION OF AN INGESTIBLE DEVICE**

(71) Applicant: Stichting IMEC Nederland, 6708 WH Wageningen (NL)
(72) Inventor: TORFS, Tom, 3080 Tervuren (BE); CASTRO MILLER, Ivan Dario, 9050 Gentbrugge (BE); ADAMOPOULOS, Vasileios, 3000 Leuven (BE); EVEN, Aniek, 6814 DT Arnhem (NL); LEONARDI, Francesca, 3901 RB Veenendaal (NL)
(74) Representative: AWA Sweden AB

(57) **Abstract**

According to an aspect of the present inventive concept there is provided an ingestible device for analysis of the gastrointestinal tract, the ingestible device comprising: at least one sensor configured for sensing a biochemical parameter, wherein the at least one sensor is configured to generate biochemical parameter data for identifying an anatomical localization of the ingestible device in the gastrointestinal tract, and a magnetic field sensor configured to receive an alternating magnetic field for generating spatial location data representing a spatial localization of the ingestible device, and wherein the ingestible device is configured to provide a combined localization of the ingestible device in the gastrointestinal tract based on the anatomical localization and the spatial localization.

## Description

### Technical field

The present description relates to an ingestible device for analysis of the gastrointestinal tract, a wearable device for localization of an ingestible device, a system for analysis of the gastrointestinal tract and a method for analysis of the gastrointestinal tract.

### Background

Ingestible devices are largely promising for analysis of the gastrointestinal tract. They may be used for analysis of the status of the gastrointestinal tract or for dispense of medicine. Ingestible devices may be a small device which may be swallowed by a person. The device may follow the gastrointestinal tract from the mouth until it is expelled naturally.

While travelling through the gastrointestinal tract, the ingestible device may receive data about the gastrointestinal tract or the ingestible device may dispense medicine. For this, the location of the ingestible device inside the gastrointestinal device is of importance. Thus, there is a need in the art for improvements.

### Summary

An objective of the present description is to provide an ingestible device for analysis of the gastrointestinal tract. If is a further objective to provide an ingestible device for analysis of the gastrointestinal tract which may provide accurate localization of the device inside the gastrointestinal tract. It is a further objective to provide a wearable device for localization of an ingestible device. It is a further objective to provide a wearable device with a highly accurate localization of an ingestible device. It is a further objective to provide a system for analysis of the gastrointestinal tract. It is a further objective to provide a method for analysis of the gastrointestinal tract.

These and other objectives are at least partly met by the invention as defined in the independent claims. Preferred embodiments are set out in the dependent claims.

According to a first aspect, there is provided an ingestible device for analysis of the gastrointestinal tract, the ingestible device comprising: at least one sensor configured for sensing a biochemical parameter, wherein the at least one sensor is configured to generate biochemical parameter data for identifying an anatomical localization of the ingestible device in the gastrointestinal tract; and a magnetic field sensor configured to receive an alternating magnetic field for generating spatial location data representing a spatial localization of the ingestible device, and wherein the ingestible device is configured to provide a combined localization of the ingestible device in the gastrointestinal tract based on the anatomical localization and the spatial localization.

By the term "gastrointestinal tract" is here meant a gastrointestinal tract of a subject. The subject may be a human and thus the gastrointestinal tract may be part of the human body. However, it is equally conceivable that the subject is an animal with a gastrointestinal tract. Therefore, it should be realized that the ingestible device may be applicable to analyze the gastrointestinal tract of any creature having a gastrointestinal tract.

The ingestible device may be an ingestible device in the form of a pill. The ingestible device should be understood as a device which can be ingested by a subject and transition through the gastrointestinal tracts without being damaged. Thus, any electronics inside of the ingestible device should not be damaged by the internal environment of the gastrointestinal tract. Moreover, the ingestible device may be collected after the transition through the gastrointestinal tract. Thus, the ingestible device may be configured for transmittance of information during the transition through the gastrointestinal tracts, it may be configured for saving information until exit of the ingestible device or a combination of both.

The ingestible device may provide a noninvasive device for measuring the status of the gastrointestinal tract. Thus, certain conditions of the gastrointestinal tract may be identified using the ingestible device, without the use of any invasive methods. This may further provide a device allowing for a cheap measurement of the gastrointestinal tract.

Providing the combined localization of the ingestible device in the gastrointestinal tract based on the anatomical localization and the spatial localization has the advantage that despite complicated anatomical structure of the gastrointestinal tract, a precise location may be provided. As such, the shape of the gastrointestinal tract may make it hard to decide the anatomical localization if only an indication of the spatial localization is available. Vice versa, only having the anatomical localization may limit the knowledge of the position into a long part of the gastrointestinal tract. As an example, the small intestine and the large intestine are at many locations spatially directly next to each other, but for the ingestible device to reach from one point to another through the gastrointestinal tract would need it to travel far in the gastrointestinal tract.

The sensor may be any sensor, such as a device, unit, circuit or element configured for sensing a biochemical parameter. The biochemical parameter may be any biochemical parameter which is measurable in the gastrointestinal tract. Thus, the biochemical parameter may be the pH, the oxidation-redox potential, the presence of ammonium or the presence of any other compound in the gastrointestinal tract.

The sensor is configured to generate biochemical parameter data for identifying an anatomical localization of the ingestible device in the gastrointestinal tract. The anatomical localization may be a region or part of the gastrointestinal tract. The anatomical localization may further be a more precise position within the gastrointestinal tract. The anatomical localization may be identified by the biochemical parameter data thanks to the changing environment in the gastrointestinal tract. Thus, a specific localization in the gastrointestinal tract may be identified by the biochemical parameter sensed by the sensor.

The biochemical parameter data may comprise a representation of a transition from a first part of the gastrointestinal tract to a second part of the gastrointestinal tract. Said transition may provide an anatomical localization of the ingestible device. Thus, the biochemical parameter data may reveal a change in the biochemical parameter data. This change may represent a transition from one part of the gastrointestinal tract to another. For instance, it may represent the transition from the stomach to the small intestine or from the small intestine to the colon.

The ingestible device further comprises a magnetic field sensor. The magnetic field sensor may comprise a magnetic field sensing coil. The magnetic field sensor is configured to receive an alternating magnetic field for generating spatial location data representing a spatial localization of the ingestible device.

An alternating magnetic field may be generated outside of the subject which has ingested the ingestible device. The magnetic field sensor is configured to measure such an alternating magnetic field. The magnetic field sensor may measure the magnitude of the magnetic field and/or the relative sign, e.g. by using the signal phase of the magnetic field received from the magnetic transmitter.

A time synchronization may be performed between any electronics in the ingestible and any electronics in the transmitter of the magnetic field.

From the magnetic field magnitude and/or sign information, from the magnetic field sensor, the spatial location data of the ingestible device in the gastrointestinal tract may be calculated. It should be understood that the spatial location data may be further improved by having the magnetic field magnitude and/or sign information from the magnetic field sensor combined with information from the transmitter such as the current used for the field generation. The calculation may be made using computer-based models of the expected generated magnetic fields and the measured magnetic field magnitudes and signs, extracted via a clustering algorithm, such as e.g. via K-means clustering from the phase information. Calculation may be performed in a processor receiving measurement information, such as a processor arranged in a device for transmitting the alternating magnetic field. Measurements by the magnetic field sensor may be transmitted to a processor arranged in any device, such as transmitting information through a computer and/or telecommunication network, e.g., to a processor "in the cloud".

The spatial location data may give a spatial location of the ingestible device having an error margin of less than 5 cm, such as less than 4 cm, such as less than 3 cm, such as less than 2 cm, such as less than 1 cm.

The ingestible device is configured to provide a combined localization of the ingestible device in the gastrointestinal tract based on the anatomical localization and the spatial localization.

It is a realization according to the first aspect that the combined localization, based on the anatomical localization and the spatial localization, gives an accurate localization of the ingestible device during transition inside the gastrointestinal tract. The localization gives both the spatial localization from the magnetic field and the anatomical localization from the biochemical parameter data. As part of the gastrointestinal tract, such as the intestine, has a serpentine like shape, having only the spatial localization would give a hint on the 3-dimensional localization of the ingestible device. However, it would still lack the more precise information of in what anatomical part of the gastrointestinal tract the ingestible device is present. For example, in some parts of the GI tract, the small intestine and the large intestine are adjacent to each other. Thus, relying only on the spatial localization, it may not be clear whether the ingestible device is in the small intestine or the large intestine. However, by using anatomical localization in addition to spatial localization, the position of the ingestible device is accurately determined as the values or change in the values of the biochemical parameter in the small intestine is different from the large intestine.

For several applications, such as identifications of conditions of the gastrointestinal tract or delivery of drugs within the gastrointestinal tract, a more precise localization may be required. In the same way, only having the anatomical localization would only indicate roughly in what part of the gastrointestinal tract the ingestible device is localized, but the more specific positioning within that part (where the biochemical parameter data would be identical) is lacking.

It is an advantage according to the present first aspect that the combined localization provides easy 3-dimensional localization of an ingestible device, not requiring medical imaging or other large medical devices. In other words, it is an advantage that the localization of the ingestible device according to the first aspect may be made with small equipment allowing for an out-of-hospital localization of the ingestible device.

From the combined localization, a time resolved 3-dimensional information of the localization of the ingestible device may be acquired. Thus, a trajectory of the transition of the ingestible device through the gastrointestinal tract may be acquired. From this trajectory, future transitions of a similar or the same ingestible device may be predicted. This may be used to investigate the lapse of a condition in the gastrointestinal tract or to administer drugs during the transition through the gastrointestinal tract. Thus, for each subject, a profile of the gastrointestinal tract may be calculated. The profile may be followed over time and changes may be registered.

According to one embodiment, the at least one sensor may comprise a pH sensor. Thus, the pH may be used as the biochemical parameter data. The pH of the gastrointestinal tracts varies throughout the tract and may therefore be used for identifying an anatomical localization.

For instance, at a transition from the stomach to the small intestine, an increase in pH may occur. Further, at a transition from the small intestine to the large intestine, a decrease may occur followed by a slow increase. Thus, by identifying a change in the pH, the anatomical localization of the gastrointestinal tract may be identified.

An advantage of this is that there may be a clear distinction between the different parts of the gastrointestinal tract. Thus, measuring the pH may allow for a good marker for a transition from a first part of the gastrointestinal tract to a second part of the gastrointestinal tract.

According to one embodiment, the at least one sensor may comprise an oxidation-reduction potential sensor (ORP).

A healthy subject typically features an anaerobic condition, especially in the distal part of the gastrointestinal tract. However, an unhealthy gut state associated with for example IBS, IBD, gastrointestinal cancer, ulcers, diabetes, ischemia, celiac disease, microbiome dysbiosis, small intestinal bacterial overgrowth (SIBO), aging, neurodegenerative diseases, cardiovascular diseases, metabolic diseases, and chronic infections may increase the level of reactive oxidizing species (e.g. superoxide, hydroxyl hypochlorous acid, and hydrogen peroxide) at the location of the unhealthy gut state. Said increase may be detected with an oxidation reduction potential (ORP) sensor. ORP is also sometimes referred to as the redox potential. ORP is measured in Volt, however the value is linked to the concentration of reducing and oxidizing species. Thus, at the location of the unhealthy gut state, a large increase in ORP values may be expected due to the presence of reactive species.

Given only as non-limiting examples, a healthy not-inflamed gastrointestinal tract may have an ORP value of between -500 mV and -300 mV in the large intestine. Thus, an ORP value of >-300 mV may serve as an indication of a possible occurrence of an unhealthy gut state in the large intestine. Given as further non-limiting examples, a healthy stomach may have an ORP value of between 50 mV and 250 mV while a healthy small intestine may have an ORP value of between 50 mV and -400 mV.

An advantage with this embodiment is that ORP may serve as a good marker for an unhealthy gut state. Thus, the anatomical localization may be combined with a marker for the health of the gastrointestinal tract.

Another advantage is that ORP may also serve as an indication of the transition between the first and second parts of the gastrointestinal tract. By way of example, the ORP value may decrease at the transition from the small intestine to the large intestine. In particular in combination with the pH, which may show a decrease followed by a slow increase at the transition from the small intestine to the large intestine, the transition may be more accurately identified. Thus, in an embodiment, the ingestible device may comprise a pH sensor and a ORP sensor.

It serves to mention that ORP may also serve as an indication of other transitions, such as from the small intestine to the large intestine, or transitions between sub-parts of the gastrointestinal tract.

It serves to mention that ORP is one way of measuring the concentration of reactive reducing and oxidizing species. However, it is conceivable that the concentration of reactive species, such as reactive oxygen species, may be measured also in other manners.

According to one embodiment, the ingestible device may further comprise an additional sensor for measuring an additional biochemical parameter.

The additional sensor may measure ammonium, or it may be a sensor for measuring a neutrophil activation marker, such as calprotectin, lactoferrin, myeloperoxidase, defensins, or S100A12. By way of further example, the additional biochemical parameter may be a cytokine, such as IL-6, IL-12, IL-23, or TNF-alpha. By way of further example, the additional biochemical parameter may be nitric oxide, matrix metalloproteinases, lysozyme, or hemoglobin. The additional biochemical parameter may also be hemoglobin, or pathogen- or microbiome-related markers.

An advantage of this is that a plurality of biochemical parameters may be used for the anatomical localization.

According to one embodiment, the at least one sensor may be configured to sense the biochemical parameter for identifying a condition of the gastrointestinal tract.

The condition of the gastrointestinal tract may be an unhealthy condition. It may as well be a healthy condition, for instance if analysis is made over time, the ingestible device may be used to measure the condition over time, wherein the condition is transferred from being unhealthy into being a healthy condition.

The condition may be an inflammatory state, or other microbiome dysbiosis. The condition may be for example IBS, IBD, gastrointestinal cancer, ulcers, diabetes, ischemia, celiac disease, microbiome dysbiosis, small intestinal bacterial overgrowth (SIBO), aging, neurodegenerative diseases, cardiovascular diseases, metabolic diseases, and chronic infections may increase the level of reactive oxidizing species (e.g. superoxide, hydroxyl hypochlorous acid, and hydrogen peroxide).

The conditions may be such that they are measurable using a biochemical parameter. Thus, the condition may be identified by a change in or the appearance of a biochemical parameter. For instance, an unwanted increase in pH or ORP may indicate the presence of a condition.

It should be noted that the condition may be identified in location and by the change of the biochemical parameter. It serves to mention that when identifying a condition in the present disclosure, no diagnosis is made, but rather an identification of the occurrence of measurement values deviant from those expected from a healthy gastrointestinal tract. However, a medical doctor provided with the data may make an assessment based on these deviant values, and thereafter make a diagnosis. Hence, the ingestible device is not a diagnostic device, but rather a device for analyzing measurement data for determining an intermediate result that may be used in diagnosis.

The at least one sensor may for instance use the biochemical parameter data for both the anatomical localization and the identification of a condition at that anatomical position.

An advantage of this is that the status of the gastrointestinal tract may be identified and also the localization of any condition may be well defined using the ingestible device according to this embodiment.

Another advantage is that it allows for identification of a condition at an early stage. This may in turn aid in promptly starting the correct therapy.

If the ingestible device comprises an additional sensor for measuring an additional biochemical parameter, the additional sensor may provide with information on the condition while the at least one sensor provides with biochemical parameter data for the anatomical localization. Alternatively, both of the sensors may provide with biochemical parameter data for the anatomical localization as well as information about a condition.

According to one embodiment, the ingestible device may further comprise a temperature sensor, wherein the temperature sensor is configured to generate temperature data at least for identifying if the ingestible device has left the gastrointestinal tract of the occurrence of a condition of the gastrointestinal tract.

The temperature sensor may be used to acquire temperature data throughout the gastrointestinal tract. The temperature sensor may further be used to acquire data on when the ingestible device has left the gastrointestinal tract. When exiting the gastrointestinal tract, the temperature will decrease. This may give a fast indication of the exit of the ingestible device.

Alternatively, or additionally, the temperature sensor may comprise information indicative of an occurrence of a condition of the gastrointestinal tract. Thus, temperature data acquired from the temperature sensor may be used to detect the occurrence of a condition. By way of example, the temperature data may show a change in value, as for example a temperature increase, at the place of the condition. An advantage is that, when analyzing temperature data in combination with data from the at least one sensor configured for sensing a biochemical parameter, an occurrence of the condition may be identified more precisely and accurately.

According to one embodiment, the ingestible device may further comprise a mechanical sensor configured to correcting disturbing relative motions between the transmittance of the alternating magnetic field and the ingestible device independent of the movement along the gastrointestinal tract and/or configured to generate an additional input to the combined localization..

The mechanical sensor may be accelerometers, gyroscopes or magnetometers. The mechanical sensor may aid in correcting for disturbing relative motions between the magnetic transmitter and the ingestible device independent of the movement along the gastrointestinal tract and/or as an additional input to the localization algorithms.

The mechanical sensor may further give an indication of the presence of a condition, as the condition may interfere with the movement through the gastrointestinal tract.

According to one embodiment, the ingestible device may further comprise a wireless communication system for communication with an external device.

The communication system may be a radio frequency transceiver, or modulation of the magnetic fields, or ultrasound communication, or conductive communication.

The communication system may be used to communicate the anatomical localization and the spatial localization in real time. Further, the communication system may be used to communicate any further biochemical data, any temperature data or any mechanical data.

According to one embodiment, the ingestible device may further comprise an energy source.

The energy source may allow for the ingestible device to be arranged within the gastrointestinal tract for a longer time. Thus, the energy source may be adapted to the use of the ingestible device.

The energy source may be a battery. Alternatively, the energy source may be a circuitry configured to receive remote wireless powering or the energy source may be configured for local energy harvesting such as a bio fuel cell.

According to one embodiment, the magnetic field sensor may comprise a plurality of magnetic field sensors.

An advantage of this is that having a plurality of magnetic field sensors may improve the spatial localization data such that measuring errors are reduced.

According to a second aspect, there is provided a wearable device for localization of an ingestible device, the wearable device comprising: a magnetic field transmitter configured for generating an alternating magnetic field to be received by a magnetic field sensor comprised in the ingestible device, the magnetic field is configured for generating spatial location data representing a spatial localization of the ingestible device while the ingestible device passes through the gastrointestinal tract; a receiving sensor configured for receiving information of the spatial localization of the ingestible device from the magnitude and relative sign of the magnetic field received by the magnetic field sensor and further configured for receiving the anatomical localization from biochemical parameter data acquired by the ingestible device while the ingestible device passes through the gastrointestinal tract; wherein the wearable device is configured to calculate a combined localization of the ingestible device in the gastrointestinal tract based on the anatomical localization and the spatial localization.

This aspect may generally present the same or corresponding advantages as the former aspect.

The wearable device may be in the form of a belt configured for being worn around the waist or the chest. Thus, the wearable device may be configured for being worn during the time when the ingestible device passes through the gastrointestinal tract.

However, it should be understood that it is not a requirement for the passing of the ingestible device through the gastrointestinal tract that the wearable device is worn at all times.

The magnetic field transmitter is configured for generating an alternating magnetic field. The magnetic field is received by a magnetic field sensor comprised in the ingestible device. The magnetic field is used for generating spatial location data representing a spatial localization of the ingestible device while the ingestible device passes through the gastrointestinal tract.

According to an embodiment of the second aspect, the wearable device may comprise several magnetic field transmitters, such as two or three magnetic field transmitters. Accordingly, the ingestible device may comprise several magnetic field sensors, such as two or three magnetic field sensors. Having several magnetic field transmitters and/or several magnetic field sensors may improve the spatial localization data such that measuring errors are reduced.

Alternating magnetic fields are generated by the magnetic field transmitter. These magnetic fields are measured by the ingestible using the magnetic field sensor. Both the magnitude of the magnetic field and its relative sign e.g by using the signal phase of the magnetic field received from several magnetic transmitters may be measured. A magnetic field measurement may use a time synchronization between electronics in the ingestible device and the electronics in the wearable device. The resulting magnitudes and/or relative signs of the magnetic field may be wirelessly transmitted from the ingestible to the wearable system by the receiving sensor. Further, the receiving sensor is further configured for receiving the anatomical localization from biochemical parameter data acquired by the ingestible device while the ingestible device passes through the gastrointestinal tract. The biochemical parameter data may be acquired by an ORP sensor, pH sensor, other biochemical sensors or temperature sensor.

The wearable device is configured to calculate a combined localization of the ingestible device in the gastrointestinal tract based on the anatomical localization and the spatial localization.

It should be understood that the calculation may be made within the wearable device, alternatively the anatomical localization and the spatial localization may be transferred to an external device for calculating the combined localization.

The biochemical parameter data may comprise a representation of a transition from a first part of the gastrointestinal tract to a second part of the gastrointestinal tract. Said transition providing an anatomical localization of the ingestible device.

According to a third aspect, there is provided a system for analysis of the gastrointestinal tract, the system comprising: an ingestible device according to the first aspect; a magnetic field transmitter configured for generating an alternating magnetic field to be sensed by the magnetic field sensor.

This aspect may generally present the same or corresponding advantages as the former aspects.

Alternating magnetic fields are generated by the magnetic field transmitter. These magnetic fields are measured by the ingestible using the magnetic field sensor. Both the magnitude of the magnetic field and its relative sign e.g. by using the phase may be measured. A magnetic field measurement may use a time synchronization between electronics in the ingestible device and the electronics in the wearable device. The resulting magnitudes and relative signs of the magnetic field may be wirelessly transmitted from the ingestible to a receiving sensor. Further, a receiving sensor may further be configured for receiving anatomical localization from biochemical parameter data acquired by the ingestible device while the ingestible device passes through the gastrointestinal tract. The biochemical parameter data may be acquired by an ORP sensor, pH sensor, other biochemical sensors or temperature sensor.

An advantage of this system is that the magnetic field may provide specific spatial localization data.

According to one embodiment of the third aspect, the system may further comprise an exit module, the exit module may comprise a permanent magnet configured for providing information whether the ingestible device is in the close proximity to the exit module.

The exit module may be placed close to the rectum at regular intervals, for example just before toilet visits. The magnetic field from the permanent magnet in the exit module can then be picked up by the magnetic field sensor comprised in the ingestible device. The closer the ingestible device is to the exit prediction module, the stronger the magnetic field measured by the magnetic field sensor in the ingestible device will be, and the more likely that the ingestible will be excreted during the next toilet visit.

An advantage of this is that it provides with a simple way of knowing when the ingestible device has left the subject. This is an advantage as waste in the sewage system may be reduced and the electronics of the ingestible device may be reused.

Further, if the ingestible device stores the data during the transportation through the gastrointestinal tract without sending it to an external device, the data must be collected from the ingestible device and thereby the recovery of the ingestible device is highly important.

Even further, the ingestible device may collect samples, such as fluids from the gastrointestinal tract, which may be recovered from the ingestible device after exit before an analysis may be made.

Using the exit module provides with a way to in advance predict when the exit is about to happen. This reduces the need for any control measures to be made blindly.

The ingestible device may further comprise a temperature sensor. The temperature sensor may complement the exit module with the prediction/confirmation on when the ingestible device has left the body.

As long as the ingestible device is inside the gastrointestinal tract, the temperature data will show the internal body temperature of the subject. In case the subject is a human, the temperature is typically around 37°C. However, upon the ingestible device exiting the body it is expected to cool down to room temperature, as for example around 20°C to 25°C. Thus, by identifying a temperature decrease from the temperature sensor, the exit time point may be determined.

According to a fourth aspect, there is provided a method for analysis of the gastrointestinal tract, the method comprising: receiving a biochemical parameter from the ingestible device, while the ingestible device passes through the gastrointestinal tract, said biochemical parameter providing an anatomical localization of the ingestible device; receiving information of the magnitude and relative sign of an alternating magnetic field from a magnetic field sensor comprised in an ingestible device, while the ingestible device passes through the gastrointestinal tract, wherein the information of the magnitude and relative sign of the alternating magnetic field comprises spatial location data representing a spatial localization of the ingestible device; determine a combined localization of the ingestible device in the gastrointestinal tract based on the anatomical localization and the spatial localization.

This aspect may generally present the same or corresponding advantages as the former aspects.

The biochemical parameter may comprise the measurement of the pH, the oxidation-reduction potential, ammonium, neutrophil activation marker, such as calprotectin, lactoferrin, myeloperoxidase, defensins, or S100A12, cytokine, such as IL-6, IL-12, IL-23, or TNF-alpha, nitric oxide, matrix metalloproteinases, lysozyme, or hemoglobin, or pathogen- or microbiome-related markers.

The biochemical parameter data may comprise a representation of a transition from a first part of the gastrointestinal tract to a second part of the gastrointestinal tract. Said transition providing an anatomical localization of the ingestible device. Thus, the biochemical parameter data may reveal a change in the biochemical parameter data. This change may represent a transition from one part of the gastrointestinal tract to another. For instance, it may represent the transition from the stomach to the small intestine or from the small intestine to the colon.

The alternating magnetic field may be generated outside of the subject which has ingested the ingestible device. The magnetic field sensor is configured for measure such a magnetic field. The magnetic field sensor may measure the magnitude of the magnetic field and/or the relative sign e.g. by using the signal phase of the magnetic field received from several magnetic transmitters.

A time synchronization may be performed between any electronics in the ingestible and any electronics in the transmitter of the magnetic field.

From the magnetic field magnitude and/or sign information, from the magnetic field sensor, the spatial location data of the ingestible device in the gastrointestinal tract may be calculated. It should be understood that the spatial location data may be further improved by having the magnetic field magnitude and/or sign information from both the magnetic field sensor and a magnetic field transmitter. The calculation may be made using computer-based models of the expected generated magnetic fields and the measured magnetic field magnitudes and signs, extracted via a clustering algorithm, such as e.g. via K-means clustering from the phase information. Calculation may be performed in a processor receiving measurement information, such as a processor arranged in a device for transmitting the alternating magnetic field. Measurements by the magnetic field sensor may be transmitted to a processor arranged in any device, such as transmitting information through a computer and/or telecommunication network, e.g., to a processor "in the cloud".

Determining the combined localization of the ingestible device in the gastrointestinal tract based on the anatomical localization and the spatial localization has the advantage that the despite complicated anatomical structure of the gastrointestinal tract, a precise location may be determined. As such, the S-shape of part of the gastrointestinal tract, such as the intestines, may make it hard to decide the anatomical localization if only an indication of the spatial localization is available. Vice versa, only having the anatomical localization may limit the knowledge of the position into a long part of the gastrointestinal tract.

The method may be performed using a processing unit. The processing unit may be implemented as a general-purpose processing unit, such as a central processing unit (CPU), which may execute the instructions of one or more computer programs in order to implement the method. The processing unit may alternatively be implemented as firmware arranged in an embedded system, or as a specifically designed processing unit, such as an Application-Specific Integrated Circuit (ASIC) or a Field-Programmable Gate Array (FPGA), which may be configured to implement the method.

According to one embodiment of the fourth aspect, the method may further comprise: receiving information from an exit module, the exit module may comprise a permanent magnet configured for providing information whether the ingestible device is in the close proximity to the exit module; determining based on the information from the exit module whether the ingestible device is expected to leave the gastrointestinal tract within a threshold time.

The exit module may be placed close to the rectum at regular intervals, for example just before toilet visits. The magnetic field from the permanent magnet in the exit module can then be picked up by the magnetic field sensor comprised in the ingestible device. The closer the ingestible device is to the exit prediction module, the stronger the magnetic field measured by the magnetic field sensor in the ingestible device will be, and the more likely that the ingestible will be excreted during the next toilet visit.

The exit module may then predict whether the ingestible device is expected to leave the gastrointestinal tract within a threshold time. The threshold time could for instance be the average time between two toilets visits, such that the exit module predicts that the ingestible device is to be expected to leave the gastrointestinal tract at the next toilet visit. Alternatively, the prediction may be such that the exit module predicts that there is a certain time until the ingestible device reaches the end of the gastrointestinal tract.

An advantage of this is that it provides with a simple way of knowing when the ingestible device has left the subject. This is an advantage as waste in the sewage system may be reduced and the electronics of the ingestible device may be reused.

Further, if the ingestible device stores the data during the transportation through the gastrointestinal tract without sending it to an external device, the data must be collected form the ingestible device and thereby the recovery of the ingestible device is highly important.

Using the exit module provides with a way to in advance predict when the exit is about to happen. This reduces the need for any control measures to be made blindly.

The ingestible device may further comprise a temperature sensor. The temperature sensor may complement the exit module with the prediction/confirmation on when the ingestible device has left the body. Thus, the receiving information from an exit module may further comprise receiving temperature data from the ingestible device.

As long as the ingestible device is inside the gastrointestinal tract, the temperature data will show the internal body temperature of the subject. In case the subject is a human, the temperature is typically around 37°C. However, upon the ingestible device exiting the body it is expected to cool down to room temperature, as for example around 20°C to 25°C. Thus, by identifying a temperature decrease from the temperature sensor, the exit time point may be determined.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1 is an ingestible device for analysis of the gastrointestinal tract.
Fig. 2 is a wearable device for localization of an ingestible device.
Fig. 3 is a system for analysis of the gastrointestinal tract.
Fig. 4 is a schematic block diagram shortly summarizing the method for analysis of the gastrointestinal tract.

### Detailed description

In cooperation with attached drawings, the technical contents and detailed description of the present inventive concept are described thereinafter according to a preferable embodiment, being not used to limit the claimed scope. This inventive concept may be embodied in many different forms and should not be construed as limited to the embodiments set forth herein; rather, these embodiments are provided for thoroughness and completeness, and fully convey the scope of the inventive concept to the skilled person.

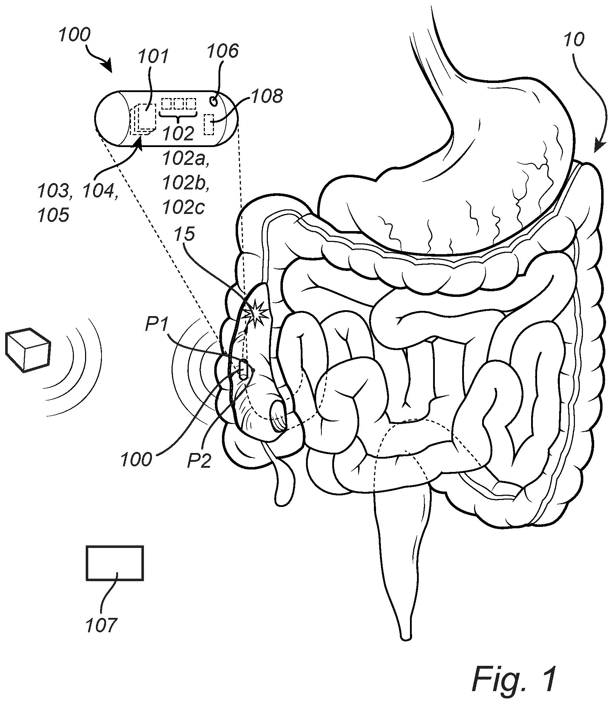

Fig. 1 illustrates an ingestible device 100 for analysis of the gastrointestinal tract 10. The ingestible device 100 may be provided in the form of a capsule or a pill. The ingestible device 100 may thus be ingested by a subject, after which the ingestible device 100 may be passed through the gastrointestinal tract 10.

The ingestible device 100 comprises at least one sensor 101 configured for sensing a biochemical parameter. The sensor 101 may be any sensor, such as a device, unit, circuit or element configured for sensing a biochemical parameter. The biochemical parameter may be any biochemical parameter which is measurable in the gastrointestinal tract 10. Thus, the biochemical parameter may be the pH, the oxidation-redox potential, the presence of ammonium or the presence of any other compound in the gastrointestinal tract 10. In other words, the sensor 101 may for instance comprise a pH sensor or an oxidation-reduction potential (ORP) sensor.

The at least one sensor 101 is configured to generate biochemical parameter data for identifying an anatomical localization P1 of the ingestible device 100 in the gastrointestinal tract 10. Thus, using the biochemical parameter may be translated into a biochemical parameter data. The anatomical localization P1 may be a region or part of the gastrointestinal tract 10. The anatomical localization P1 may further be a more precise localization within the gastrointestinal tract 10. The anatomical localization P1 may be identified by the biochemical parameter data thanks to the changing environment in the gastrointestinal tract 10. Thus, a specific localization in the gastrointestinal tract 10 may be identified by the biochemical parameter sensed by the sensor 101.

The biochemical parameter data may comprise a representation of a transition from a first part of the gastrointestinal tract 10 to a second part of the gastrointestinal tract 10. Said transition may provide an anatomical localization P1 of the ingestible device 100. Thus, the biochemical parameter data may reveal a change in the biochemical parameter data. This change may represent a transition from one part of the gastrointestinal tract 10 to another. For instance, it may represent the transition from the stomach to the small intestine or from the small intestine to the colon.

The ingestible device 100 may further comprise an additional sensor 103 for measuring an additional biochemical parameter. The additional sensor 103 may measure ammonium, or it may be a sensor 103 for measuring a neutrophil activation marker, such as calprotectin, lactoferrin, myeloperoxidase, defensins, or S100A12. By way of further example, the additional biochemical parameter may be a cytokine, such as IL-6, IL-12, IL-23, or TNF-alpha. By way of further example, the additional biochemical parameter may be nitric oxide, matrix metalloproteinases, lysozyme, or hemoglobin. The additional biochemical parameter may also be hemoglobin, or pathogen- or microbiome-related markers.

The at least one sensor 101, alternatively or additionally the additional sensor 103, may be configured to sense the biochemical parameter for identifying a condition 15 of the gastrointestinal tract 10. The condition 15 of the gastrointestinal tract may be an unhealthy condition 15. It may as well be a healthy condition 15, for instance if analysis is made over time, the ingestible device may be used to measure the condition 15 over time, wherein the condition 15 is transferred from being unhealthy into being a healthy condition.

The condition 15 may be inflammation, IBS, IBD, gastrointestinal cancer, ulcers, diabetes, ischemia, celiac disease, microbiome dysbiosis, small intestinal bacterial overgrowth (SIBO), aging, neurodegenerative diseases, cardiovascular diseases, metabolic diseases, or chronic infections.

The conditions 15 may be such that they are measurable using a biochemical parameter.

The ingestible device 100 further comprises a magnetic field sensor 102. As illustrated in Fig 1, the magnetic field sensor 102 may comprise a plurality of magnetic field sensors 102a, 102b, 102c.

The magnetic field sensor 102 is configured to receive an alternating magnetic field for generating spatial location data representing a spatial localization P2 of the ingestible device 100.

The magnetic field sensor 102 may comprise a magnetic field sensing coil. The magnetic field sensor 102 configured to receive an alternating magnetic field for generating spatial location data representing a spatial localization P2 of the ingestible device.

An alternating magnetic field may be generated outside of the subject which has ingested the ingestible device 100. As illustrated in Fig. 1 there is a magnetic field transmitter located outside of the subject. The magnetic field sensor is configured to measure such an alternating magnetic field. The magnetic field sensor 102 may measure the magnitude of the magnetic field and/or the relative sign e.g. by using the signal phase of the magnetic field received from several magnetic transmitters.

A time synchronization may be performed between any electronics in the ingestible device 100 and any electronics in the transmitter of the magnetic field.

From the magnetic field magnitude and sign information, the spatial location data of the ingestible device in the gastrointestinal tract may be calculated. The calculation may be made using computer-based models of the expected generated magnetic fields and the measured magnetic field magnitudes and signs, extracted via a clustering algorithm, such as e.g. via K-means clustering from the phase information. Calculation may be performed in a processor receiving measurement information, such as a processor arranged in a device for transmitting the alternating magnetic field. Measurements by the magnetic field sensor may be combined with magnetic field magnitude and sign information for the transmitter and may be transmitted to a processor arranged in any device, such as transmitting information through a computer and/or telecommunication network, e.g., to a processor "in the cloud".

The spatial localization P2 may comprise data from several magnetic field sensor 102a, 102b, 102c, allowing for even more accurate spatial localization.

The ingestible device 100 is configured to provide a combined localization P3 of the ingestible device 100 in the gastrointestinal tract 10 based on the anatomical localization P1 and the spatial localization P2.

The ingestible device 100 may further comprise a temperature sensor 104. The temperature sensor 104 may be used to acquire temperature data throughout the gastrointestinal tract 10. The temperature sensor 104 may further be used to acquire data on when the ingestible device 100 has left the gastrointestinal tract 10. When exiting the gastrointestinal tract 10, the temperature will decrease. This may give a fast indication of the exit of the ingestible device 100.

Alternatively, or additionally, the temperature sensor 104 may comprise information indicative of an occurrence of a condition of the gastrointestinal tract 10. Thus, temperature data acquired from the temperature sensor 104 may be used to detect the occurrence of a condition. By way of example, the temperature data may show a change in value, as for example a temperature increase, at the place of the condition.

The ingestible device 100 may further comprise a mechanical sensor 105.

The mechanical sensor 105 may be accelerometers, gyroscopes or magnetometers. The mechanical sensor 105 may aid in correcting for disturbing relative motions between the magnetic transmitter and the ingestible device 100 independent of the movement along the gastrointestinal tract 10 and/or as an additional input to the localization algorithms.

The mechanical sensor 105 may further give an indication of the presence of a condition, as the condition may interfere with the movement through the gastrointestinal tract.

The ingestible device 100 may further comprise a wireless communication system 106 for communication with an external device 107. The communication system 106 may be a radio frequency transceiver, or modulation of the magnetic fields, or ultrasound communication, or conductive communication. The communication system 106 may be used to communicate the anatomical localization P1 and the spatial localization P2 in real time. Further, the communication system 106 may be used to communicate any further biochemical data, any temperature data or any mechanical data.

The ingestible device 100 may further comprise an energy source 108.

The energy source 108 may allow for the ingestible device 100 to be arranged within the gastrointestinal tract 10 for a longer time. Thus, the energy source 108 may be adapted to the use of the ingestible device 100. The energy source 108 may be a battery. Alternatively, the energy source 108 may be a circuitry configured to receive remote wireless powering or the energy source may be configured for local energy harvesting such as a bio fuel cell.

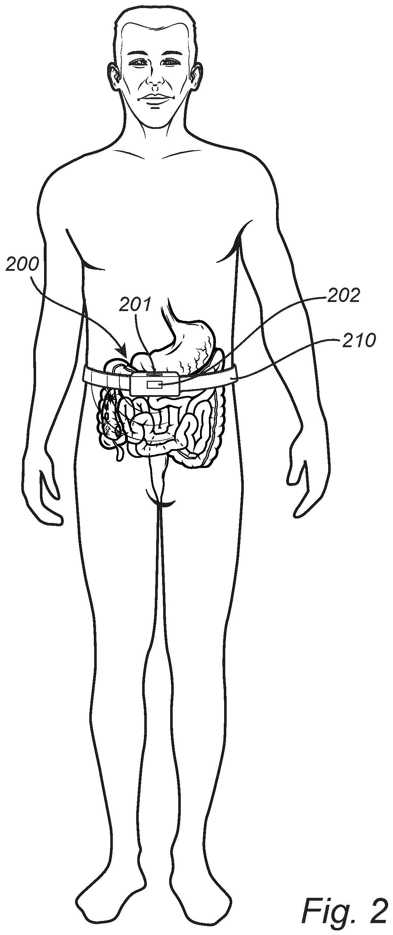

Fig. 2 illustrates a wearable device 200 for localization of an ingestible device 100. The wearable device 200 comprises a magnetic field transmitter 202. The magnetic field transmitter 202 is configured for generating an alternating magnetic field to be received by a magnetic field sensor 102. The magnetic field sensor 102 is comprised in the ingestible device 100. The ingestible device 100 may be an ingestible device 100 discloses in relation to Fig. 1. The magnetic field is configured for generating spatial location data representing a spatial localization P2 of the ingestible device 100 while the ingestible device 100 passes through the gastrointestinal tract.

The wearable device 200 further comprises a receiving sensor 201 configured for receiving information of the spatial localization P2 of the ingestible device 100 from the magnitude and sign of the magnetic field received by the magnetic field sensor 102. Further the receiving sensor 201 is configured for receiving the anatomical localization P1 from biochemical parameter data acquired by the ingestible device 100 while the ingestible device 100 passes through the gastrointestinal tract 10.

In Fig. 2 the magnetic field transmitter 202 and the receiving sensor 201 are disclosed in the same unit. However, it should be understood that the magnetic field transmitter 202 and the receiving sensor 201 may be arranged as different units.

The wearable device 200 is configured to calculate a combined localization P3 of the ingestible device 100 in the gastrointestinal tract 10 based on the anatomical localization P1 and the spatial localization P2.

The wearable device 200 may comprise a processing unit configured for localization of the ingestible device 100 when passing through the gastrointestinal tract 10, based on the biochemical data and the spatial data. As illustrated in Fig. 2, the wearable device 200, comprising the receiving sensor 201, is a device external to the sensor device 100 and external to the subject. In order for the wearable device 200 to be able to analyze the data acquired by the ingestible device 100, the ingestible device 100 is configured to provide the biochemical parameter data or the anatomical localization P1 and the spatial data or the spatial localization P2 to the wearable device 200. By way of example, the ingestible device 100 may further comprise a transmission unit 106 enabling the ingestible device 100 to wirelessly transmit the time series of data. The wirelessly transmitted data may be received by the receiving sensor 201 comprise in the wearable device 200.

The wearable device 200 may further comprise a band 210. The band 210 may be an elastic band. The receiving sensor 201 and the magnetic field transmitter 202 may be arranged on the band 210. The receiving sensor 201 and the magnetic field transmitter 202 may be arranged on the band 210 within an enclosure as is illustrated in Fig. 2. However, it should be understood that other arrangements are possible as well. The band 210 may be an adjustable band such that it may fit around the waist, the chest or the arm of the subject.

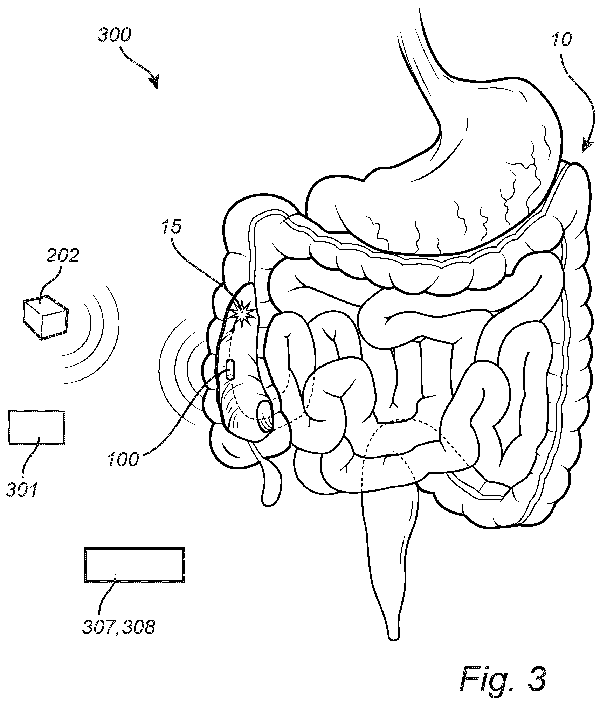

Fig. 3 illustrates a system 300 for analysis of the gastrointestinal tract 10. The system 300 comprises an ingestible device 100 according to the disclosure of Fig. 1. For reasons of simplicity, the ingestible device 100 is not disclosed again.

The system 300 further comprises a magnetic field transmitter 202. The magnetic field transmitter 202 is configured for generating an alternating magnetic field to be sensed by the magnetic field sensor 102.

The alternating magnetic field is generated by the magnetic field transmitter 202, outside of the subject which has ingested the ingestible device 100. The magnetic field sensor comprised in the ingestible device 100 is configured for measuring such a magnetic field. The magnetic field sensor 102 may measure the magnitude of the magnetic field and/or the relative sign e.g. by using the signal phase of the magnetic field received from several magnetic transmitters.

A time synchronization may be performed between any electronics in the ingestible device 100 and any electronics in the transmitter of the magnetic field 202.

From the magnetic field magnitude and sign information the spatial location data of the ingestible device 100 in the gastrointestinal tract 10 may be calculated. The calculation may be made using computer-based models of the expected generated magnetic fields and the measured magnetic field magnitudes and signs, extracted via a clustering algorithm, such as e.g. via K-means clustering from the phase information. Calculation may be performed in a processor receiving measurement information, such as a processor arranged in a device for transmitting the alternating magnetic field. Measurements by the magnetic field sensor 102 may be combined with magnetic field magnitude and sign information for the transmitter and may be transmitted to a processor arranged in any device, such as transmitting information through a computer and/or telecommunication network, e.g., to a processor "in the cloud".

The system 300 may comprise a processing unit 301 configured for analysis of the gastrointestinal tract 10, based on the biochemical data and the spatial data. The processing unit 301 may be external to the subject. In order for the processing unit 301 to be able to analyze the data acquired by the ingestible device 100, the ingestible device 100 is configured to provide the biochemical parameter data or the anatomical localization P1 and the spatial data or the spatial localization P2 to the processing unit 301. By way of example, the ingestible device 100 may further comprise a transmission unit enabling the ingestible device 100 to wirelessly transmit the biochemical parameter data or the anatomical localization P1 and the spatial data or the spatial localization P2. The wirelessly transmitted data may be received by the processing unit 301.

The system 300 may further comprise an exit module 307. The exit module 307 may comprise a permanent magnet 308 configured for providing information whether the ingestible device 100 is in the close proximity to the exit module 307.

The exit module 307 may be used to predict when the ingestible device 100 is in close proximity to the exit module 307. It may then be highly likely that the ingestible device 100 will leave the body at the next toilet visit.

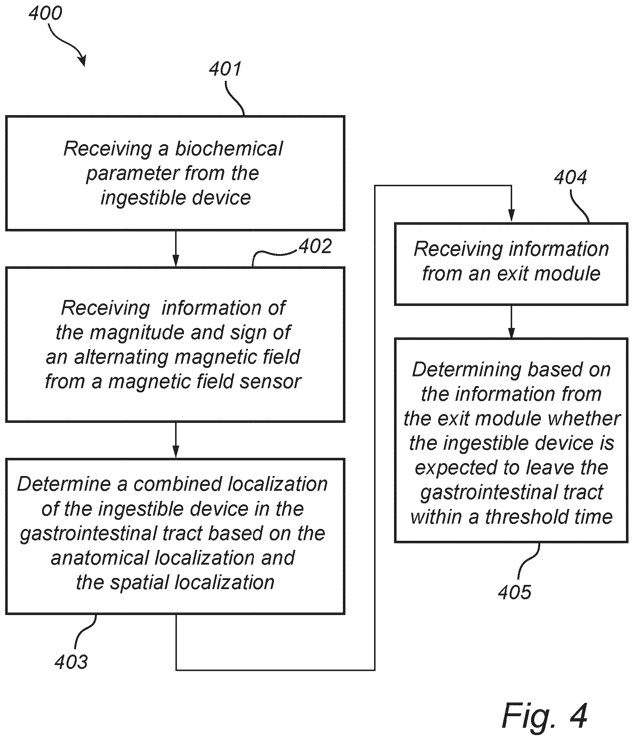

Fig. 4 illustrates a schematic representation of a method 400 for analysis of the gastrointestinal tract 10. The method 400 comprises receiving 401 a biochemical parameter from the ingestible device 100, while the ingestible device 100 passes through the gastrointestinal tract 10. Said biochemical parameter provides an anatomical localization P1 of the ingestible device 100. The biochemical parameter may be any biochemical parameter measurable in the gastrointestinal tract 10. For instance, the biochemical parameter may be the pH, the oxidation-redox potential, the presence of ammonium or the presence of any other compound in the gastrointestinal tract 10. The ingestible device 100 may comprise a sensor 101, such as for instance comprise a pH sensor or an oxidation-reduction potential (ORP) sensor.

The biochemical parameter may be translated into a biochemical parameter data. The anatomical localization P1 may be a region or part of the gastrointestinal tract 10. The anatomical localization P1 may further be a more precise localization within the gastrointestinal tract 10. The anatomical localization P1 may be identified by the biochemical parameter data thanks to the changing environment in the gastrointestinal tract 10. Thus, a specific localization in the gastrointestinal tract 10 may be identified by the biochemical parameter sensed by the sensor 101.

The biochemical parameter data may comprise a representation of a transition from a first part of the gastrointestinal tract 10 to a second part of the gastrointestinal tract 10. Said transition may provide an anatomical localization P1 of the ingestible device 100. Thus, the biochemical parameter data may reveal a change in the biochemical parameter data. This change may represent a transition from one part of the gastrointestinal tract 10 to another. For instance, it may represent the transition from the stomach to the small intestine or from the small intestine to the colon.

The biochemical parameter may further be used for identifying a condition 15 of the gastrointestinal tract 10. The condition 15 of the gastrointestinal tract may be an unhealthy condition 15. It may as well be a healthy condition 15, for instance if analysis is made over time, the ingestible device may be used to measure the condition 15 over time, wherein the condition 15 is transferred from being unhealthy into being a healthy condition.

The condition 15 may be inflammation, IBS, IBD, gastrointestinal cancer, ulcers, diabetes, ischemia, celiac disease, microbiome dysbiosis, small intestinal bacterial overgrowth (SIBO), aging, neurodegenerative diseases, cardiovascular diseases, metabolic diseases, or chronic infections.

The method 400 further comprises receiving 402 information of the magnitude and sign of an alternating magnetic field from a magnetic field sensor 102 comprised in an ingestible device 100, while the ingestible device 100 passes through the gastrointestinal tract 10.

An alternating magnetic field may be generated outside of the subject which has ingested the ingestible device 100. The magnetic field sensor 102 is configured to measure such an altering magnetic field. The magnetic field sensor 102 may measure the magnitude of the magnetic field and/or the relative sign e.g. by using the signal phase of the magnetic field received from several magnetic transmitters.

A time synchronization may be performed between any electronics in the ingestible device 100 and any electronics in the transmitter of the magnetic field 202.

The information of the magnitude and sign of the alternating magnetic field comprises spatial location data representing a spatial localization P2 of the ingestible device 100.

From the magnetic field magnitude and sign information the spatial location data of the ingestible device in the gastrointestinal tract may be calculated. The calculation may be made using computer-based models of the expected generated magnetic fields and the measured magnetic field magnitudes and signs, extracted via a clustering algorithm, such as e.g. via K-means clustering from the phase information. Calculation may be performed in a processor receiving measurement information, such as a processor arranged in a device for transmitting the alternating magnetic field. Measurements by the magnetic field sensor may be combined with magnetic field magnitude and sign information for the transmitter and may be transmitted to a processor arranged in any device, such as transmitting information through a computer and/or telecommunication network, e.g., to a processor "in the cloud".

The method 400 further comprises determine 403 a combined localization P3 of the ingestible device 100 in the gastrointestinal tract 10 based on the anatomical localization P1 and the spatial localization P2. The combined localization P3 takes into account the knowledge of the anatomical localization P1 based on the biochemical parameter data, which may tell in what part of the gastrointestinal tract the ingestible device 100 is present in and the spatial localization P2 which can tell for instance the placement of the transverse plane of the gastrointestinal tract 10 in which the ingestible device 100 is present.

The method 400 may further comprise receiving 404 information from an exit module 307. The exit module 307 may comprise a permanent magnet 308 configured for providing information whether the ingestible device 100 is in the close proximity to the exit module 307. The method 400 additionally may comprise determining 405 based on the information from the exit module 307 whether the ingestible device 100 is expected to leave the gastrointestinal tract 10 within a threshold time T. The threshold time T may for instance indicate that the ingestible device 100 may exit the subject at the next toilet visit.

The ingestible device 100 may further comprise a temperature sensor 104. The temperature sensor 104 may be used to acquire temperature data throughout the gastrointestinal tract 10. The temperature sensor 104 may further be used to acquire data on when the ingestible device 100 has left the gastrointestinal tract 10. When exiting the gastrointestinal tract 10, the temperature will decrease. This may give a fast indication of the exit of the ingestible device 100.

Thus, receiving 404 information from an exit module 307 may additionally comprise receiving temperature data from a temperature sensor 104. Thus, the exit of the ingestible device 100 may be based on information from the exit module 307, anatomical localization, spatial localization and temperature data.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. An ingestible device (100) for analysis of the gastrointestinal tract (10), the ingestible device (100) comprising:
at least one sensor (101) configured for sensing a biochemical parameter, wherein the at least one sensor (101) is configured to generate biochemical parameter data for identifying an anatomical localization (P1) of the ingestible device (100) in the gastrointestinal tract (10); and
a magnetic field sensor (102) configured to receive an alternating magnetic field for generating spatial location data representing a spatial localization (P2) of the ingestible device (100), and
wherein the ingestible device (100) is configured to provide a combined localization (P3) of the ingestible device (100) in the gastrointestinal tract (10) based on the anatomical localization (P1) and the spatial localization (P2).

2. The ingestible device (100) according to claim 1, wherein the at least one sensor (101) comprises a pH sensor.

3. The ingestible device (100) according to any one of claims 1 or 2, wherein the at least one sensor (101) comprises an oxidation-reduction potential sensor (ORP).

4. The ingestible device (100) according to any one of the preceding claims, wherein the ingestible device (100) further comprises an additional sensor (103) for measuring an additional biochemical parameter.

5. The ingestible device (100) according to any one of the preceding claims, wherein the at least one sensor (101) is configured to sense the biochemical parameter for identifying a condition (15) of the gastrointestinal tract (10).

6. The ingestible device (100) according to any one of the preceding claims, wherein the ingestible device (100) further comprises a temperature sensor (104), wherein the temperature sensor is configured to generate temperature data at least for identifying if the ingestible device (100) has left the gastrointestinal tract of the occurrence of a condition (15) of the gastrointestinal tract (10).

7. The ingestible device (100) according to any one of the preceding claims, wherein the ingestible device (100) further comprises a mechanical sensor (105) configured to correcting disturbing relative motions between the transmittance of the alternating magnetic field and the ingestible device (100) independent of the movement along the gastrointestinal tract (10) and/or configured to generate an additional input to the combined localization (P3).

8. The ingestible device (100) according to anyone of the preceding claims, wherein the ingestible device (100) further comprises a wireless communication system (106) for communication with an external device (107).

9. The ingestible device (100) according to anyone of the preceding claims, wherein the ingestible device (100) further comprises an energy source (108).

10. The ingestible device (100) according to anyone of the preceding claims, wherein the magnetic field sensor (102) comprises a plurality of magnetic field sensors (102a, 102b, 102c).

11. A wearable device (200) for localization of an ingestible device (100), the wearable device (200) comprising:
a magnetic field transmitter (202) configured for generating an alternating magnetic field to be received by a magnetic field sensor (102) comprised in the ingestible device (100), the magnetic field is configured for generating spatial location data representing a spatial localization (P2) of the ingestible device (100) while the ingestible device (100) passes through the gastrointestinal tract (10);
a receiving sensor (201) configured for receiving information of the spatial localization (P2) of the ingestible device (100) from the magnitude and relative sign of the magnetic field received by the magnetic field sensor (102) and further configured for receiving the anatomical localization (P1) from biochemical parameter data acquired by the ingestible device (100) while the ingestible device (100) passes through the gastrointestinal tract (10);
wherein the wearable device (200) is configured to calculate a combined localization (P3) of the ingestible device (100) in the gastrointestinal tract (10) based on the anatomical localization (P2) and the spatial localization (P3).

12. A system (300) for analysis of the gastrointestinal tract (10), the system (300) comprising:
an ingestible device (100) according to any of the claims 1-10;
a magnetic field transmitter (202) configured for generating an alternating magnetic field to be sensed by the magnetic field sensor (102).

13. The system (300) according to claim 12, wherein the system (300) further comprises an exit module (307), the exit module (307) comprising a permanent magnet (308) configured for providing information whether the ingestible device (100) is in the close proximity to the exit module (307).

14. A method (400) for analysis of the gastrointestinal tract, the method (400) comprising:
receiving (401) a biochemical parameter from the ingestible device (100), while the ingestible device (100) passes through the gastrointestinal tract (10), said biochemical parameter providing an anatomical localization (P1) of the ingestible device (100);
receiving (402) information of the magnitude and relative sign of an alternating magnetic field from a magnetic field sensor (102) comprised in an ingestible device (100), while the ingestible device (100) passes through the gastrointestinal tract (10), wherein the information of the magnitude and relative sign of the alternating magnetic field comprises spatial location data representing a spatial localization (P2) of the ingestible device (100);
determine (403) a combined localization (P3) of the ingestible device (100) in the gastrointestinal tract (10) based on the anatomical localization (P1) and the spatial localization (P2).

15. The method (400) according to claim 14, wherein the method (400) further comprises:
receiving (404) information from an exit module (307), the exit module (307) comprising a permanent magnet (308) configured for providing information whether the ingestible device (100) is in the close proximity to the exit module (307);
determining (405) based on the information from the exit module (307) whether the ingestible device (100) is expected to leave the gastrointestinal tract (10) within a threshold time (T).
